(19) Europäisches Patentamt
European Patent Office
Office européen des brevets

(11) **EP 3 292 876 A1**

(12) **EUROPEAN PATENT APPLICATION**

(43) Date of publication:
**14.03.2018 Bulletin 2018/11**

(51) Int Cl.:
***A61K 49/08*** *(2006.01)* ***A61K 49/18*** *(2006.01)*

(21) Application number: **16188270.9**

(22) Date of filing: **12.09.2016**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**MA MD**

(71) Applicant: **Technische Universität Graz**
**8010 Graz (AT)**

(72) Inventors:
- **Petrovic, Andreas**
  **8010 Graz (AT)**
- **Spirk, Stefan**
  **8020 Graz (AT)**
- **Scharfetter, Hermann**
  **8045 Graz (AT)**
- **Fischer, Roland**
  **8041 Graz (AT)**
- **Kruk, Danuta**
  **10-692 Olsztyn (PL)**

(74) Representative: **Patentanwaltskanzlei Matschnig & Forsthuber OG**
**Biberstraße 22**
**Postfach 36**
**1010 Wien (AT)**

# (54) COMPOUNDS FOR USE AS CONTRAST AGENTS IN MAGNETIC RESONANCE IMAGING

(57) The invention relates to a contrast agent particle comprising a carrier particle having a particle size of between 5 and 200 nm and at least one quadrupolar compound comprising at least one bismuth as a quadrupolar nucleus, wherein the at least one quadrupolar compound is bound to the carrier particle via at least one linker. The invention also relates to a Magnetic Resonance Imaging contrast medium (MRI contrast medium) comprising a contrast agent particle according to the invention. Furthermore, the invention relates to the use of contrast agent particles and Magnetic Resonance Imaging media according to the invention in Quadrupole Relaxation Enhancement (QRE)-based Magnetic Resonance Imaging.

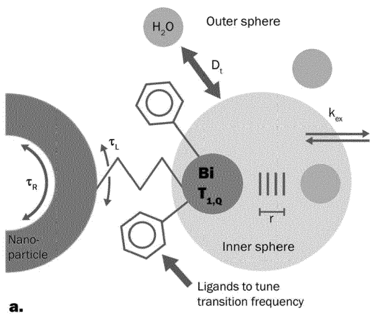


Fig. 1

Schematic view of the structure and the action mechanism of the new CAs when containing $^{209}Bi$ as the QN: Water molecules approach the Bi atom by translational diffusion ($D_t$). Protons relax with shortened T1 (at specific frequencies) due to mutual dipole-dipole coupling to QN. $k_{ex}$ symbolizes the water exchange ratio, r is the distance between proton and $^{209}Bi$. The $^{209}Bi$ -atom possesses ligands which, together with the external static magnetic field determine its quadrupole resonance transition frequencies. Bi and the ligands constitute the core compound, **component (i)**. Component 1 is grafted via a linker (zig-zag line) onto a biocompatible nanoparticle. The linker forms **component (ii)** while the nanoparticle represents c**omponent (iii)**.

EP 3 292 876 A1

## Description

The present invention relates to a contrast agent particle for use in molecular imaging, in particular Molecular Resonance Imaging (MRI). Furthermore, the invention also relates to a Magnetic Resonance Imaging contrast medium (MRI contrast medium) comprising a contrast agent particle according to the invention.

Molecular imaging (MI) is one of the key tools in medical diagnostics. It provides spatially and temporally resolved maps of relevant biomarkers and thus plays a pivotal role in the further development of personalised medicine. Amongst all medical imaging modalities, magnetic resonance imaging (MRI) is one of the most versatile and powerful due to its superior intrinsic soft tissue contrast. MRI is steadily developing towards becoming a biomarker imaging modality, with the final aim of satisfying expectations associated with MI. Certainly, MI methods other than MRI do exist but all of these suffer from significant shortcomings. PET and PET-MRI, for instance, provide the highest sensitivity but a rather low spatial resolution and require short-lived radioisotopes generated in cyclotrons and the contrast cannot be switched off after contrast agent administration. Optical methods, with the exception of photo-acoustic tomography, feature limited penetration depth and yield comparatively low resolution. X-ray CT relies on ionizing radiation and the contrast cannot be switched off. Ultrasound suffers from limited penetration depth and resolution. MRI is free of ionizing radiation, has high resolution and provides a vast number of contrast mechanisms which make it useful both for animal studies and clinical use.

However, several limitations exist in the implementation of MRI as an MI methodology, especially moderate sensitivity and difficulty in tracking several contrast agents (CAs) simultaneously. In clinical practice usually Gadolinium (Gd) based paramagnetic chelates are injected which shorten the so called MR relaxation times of the imaged protons and thus produce specific contrast. Significant effort has gone into the development of nanoparticles (NPs) containing paramagnetic iron oxides, which predominantly shorten the $T_2$ relaxation time. In the last few years, developments have led towards "smart" contrast agents that shorten the longitudinal relaxation time $T_1$ and additionally exhibit on and off switches or are sensitive to physiological parameters such as pH. Smart CAs, e.g. based on chemical exchange saturation transfer (CEST) exhibit low sensitivities and suffer from saturation spill over effects between bulk protons and protons of the CEST agent due to small frequency separations. Imaging of nuclei other than hydrogen ($^{1}H$) is an additional MI possibility, e.g. with fluorine ($^{19}F$) which exhibits good sensitivity (due to the lack of background signal) but has long $T_1$ values, leading to prolonged scan times. Imaging with hyperpolarised $^{13}C$ is a complex procedure which is only useful for short-lived MI examinations. Although paramagnetic CAs are currently believed to yield the highest possible relaxation enhancement due to the large magnetic moment of electrons, however, as a result of fast electronic relaxation, the enhancement actually achievable is far below the theoretical limit.

It is therefore an object of the present invention to provide novel "smart" contrast agents for MRI. It is another object of the present invention to provide an improved MRI contrast medium.

The present invention provides a novel contrast agent particle for MRI, which contrast agent particle is characterized in that it comprises a carrier particle having a particle size of between 5 and 200 nm and at least one quadrupolar compound comprising at least one bismuth as a quadrupolar nucleus, wherein the at least one quadrupolar compound is bound to the carrier particle via at least one linker.

The present invention also provides a novel Magnetic Resonance Imaging contrast medium (MRI contrast medium) comprising a contrast agent particle according to the invention and as described herein.

The contrast agent particle and MRI contrast medium of the invention allow for the exploitation of a new relaxation mechanism in MRI, the so called Quadrupole Relaxation enhancement (QRE) by employing a quadrupolar compound comprising at least one bismuth as a quadrupolar nucleus, wherein the quadrupolar compound is attached to a carrier particle having a particle size of between 5 and 200 nm. One important advantage which distinguishes the contrast agent particles of the invention from established ones is their strongly frequency-selective effectivity. The present invention opens a wide range of possibilities to functionalize contrast agents in terms of 'smart' action, i. e. to activate or inactivate them by external (i. e. a magnetic field) or internal signals (i. e. binding to specific sites, changes of the particle rotation or local magnetic field in the tissue by pathophysiological processes).

The contrast agent particle according to the present invention is composed of three components: at least one quadrupolar compound (i) that comprises at least one bismuth as a quadrupolar nucleus, and a linker (ii) which connects the at least one quadrupolar compound (i) with a carrier particle (iii) having a size between 5 and 200 nm (see also Fig. 1 and description thereof, showing a schematic view of the structure and action mechanism of the contrast agent particles according to the invention.).

For the component (i), bismuth compounds with quadrupolar nuclei having high nuclear spin quantum numbers together with high $\gamma_Q$ and high natural abundance are employed. The term "bismuth" as used herein refers to $^{209}Bi$. Bismuth/$^{209}Bi$ features low toxicity, particularly compared to other heavy metals and, thus, the contrast agent particles according to the invention are expected to have a higher biocompatibility than e.g. Gadolinium based CAs. Furthermore, the bismuth-containing quadrupolar compounds as used in the present invention are more diverse and hence provide the option to chemically modify and optimize the spectroscopic properties. As will be explained in more detail below, the

most preferred contrast agent particles are based on various (e.g. phenyl, naphthyl, furyl, pyridyl) arylated bismuth compounds (either mono, di or triarylated) which exhibit different substitution patterns on the rings (see embodiments below). These substituents have three functions: on the one hand they can be used to tune the NQR frequency, on the other hand they allow for a tuning of the polarity of the compound and further contain linking sites to chemically connect the bismuth compounds to the carrier particles (iii) via the linker (ii).

The linker (ii) has the role of connecting the quadrupolar compound (i) with the carrier particle (iii). For example, in optionally substituted arylated bismuth compounds as described below (see e.g. formulas (I) to (VI)), these linkers can be e.g. attached on the arylated rings. Alternatively, the linker can be anchored directly on the bismuth. The grafting of the quadrupolar compound onto the carrier particle is done at a rather large distance to the active center in order to avoid influencing the transitions of resonance frequencies. The attachment of chemical entities to carrier particles via linkers is well known in the art. The quadrupolar compound can be attached to the carrier particle covalently or non-covalently, e.g. electrostatic and/or hydrophobic interactions. Preferably, the at least one quadrupolar compound is covalently bound to the carrier particle via the at least one linker. For example, a functional group to graft the bismuth compound onto the particle could e.g. be a terminal alkyne, whereas the substituent of choice is propargyl. The terminal alkyne acts as reaction partner in a Huisgen type dipolar cycloaddition with an azide covalently attached on a carrier particle in order to provide a covalent linkage of the carrier particle and the NQR active component. In a similar manner, also an azide can be used as functional group, whereas in this case an alkyne is grafted on the carrier particle finally reacting with the azide to form a covalent bond. Generally, it is well known to those skilled in the art to match a reactive species and reactive group to undergo a reaction yielding covalently linked conjugates, which is in the present invention a contrast agent particle having a "quadrupolar compound(s)-linker-nanoparticle"-structure.

The component (iii) (carrier particle) has several functions, namely to regulate the rotational constraints of the bismuth compound attached to the particle. This regulation is required to exploit the QRE. The requirements for the nanoparticle include also to induce biocompatibility as well as to achieve sufficient stability during the measurements.

The characteristic features of the contrast agent particles according to the present invention are as follows:

> The contrast mechanism relies on the shortening of the $T_1$ relaxation time (and simultaneously also the $T_2$ time) of the proton NMR signal after the excitation of the protons in water and fat in body tissues. In contrast to established paramagnetic CAs (e.g. based on Gadolinium) the shortening is achieved by so called QRE . In the invention focus is made on bismuth, $^{209}$Bi (I=9/2), a nucleus with a high nuclear spin quantum number. Bismuth also has a comparatively high magnetogyric ratio. Bismuth has been suggested also in x-ray contrast agents [Rabin O. et al., Nat. Mater., Vol. 5, Nr. 2. P. 118-122, Feb. 2006]. US-patent US 5,817,289 suggests bismuth compounds to be used in the context of MRI contrast agents but in this document the bismuth nucleus is meant to act as an X-ray absorber in a molecule which also contains a compound which is active as MRI CA (dual modality CA). Moreover, this disclosure only refers to single molecular compounds rather than bismuth compounds which are bound to carrier particles via a linker species.

In contrast to the established paramagnetic contrast agents the QRE - based contrast mechanism is effective only in very narrow frequency bands, because the relaxation enhancement occurs only at frequencies close to the transition frequencies of nuclear quadrupole resonance (NQR).

As the transition frequencies of the bismuth depend on the bonding electrons and on the charged structures around bismuth, changes of the conformation and the distances of polar groups from the bismuth lead to frequency shifts and hence to a selectivity of the contrast to chemical interactions of the quadrupolar compound with its environment. This is the key for developing 'smart' CAs which are sensitive to chemical changes.

The fact that the bonding structure and hence the transition frequencies are sensitive to the temperature (the frequency changes typically range between several kHz up to 160 kHz per °C) opens the possibility of temperature mapping.

As strong electric fields can influence the conformation of the ligands, there is also the possibility for sensing and mapping biopotentials (e.g. membran potentials).

The NQR transition frequencies depend also on the static magnetic field Bo of the MR-scanner. Thus the QRE and hence the contrast effect can be switched on and off by changing the Bo field, which opens a very attractive possibility for differential (with respect to the applied external magnetic field and hence the QRE frequencies) imaging. Furthermore, CAs with different NQR transition frequencies can be activated selectively with different Bo and hence several pathological processes in tissue can be imaged simultaneously (,multispectral imaging'). Furthermore, $^{209}$Bi has a sufficiently high magnetogyric ratio $\gamma_Q$ ensuring the sensitivity of QRE transition frequencies to subtle changes of the magnetic field.

As it is the case for many established paramagnetic NMR-CAs, changes of the rotational dynamics of the particles strongly modulate the efficiency of the contrast. Thus pathophysiological processes that lead to changes in dynamical parameters of the tissue (like viscosity) also change the contrast.

The ligand groups attached to bismuth ($^{209}$Bi) in the quadrupolar compound can be modified to render them

hydrophobic and thus interact with lipids rather than water and allow for additional selective mapping of e.g. fat tissue.

In preferable embodiments the at least one quadrupolar compound is an optionally substituted arylated bismuth compound. Substitution of the aryl ring(s) allows for fine tuning the resonance frequency. Preferably, one or more of the aryl rings of the quadrupolar bismuth compound are substituted at their C2-position. The linker is preferably attached to C4 of one or more of the aryl rings; this allows for anchoring the optionally substituted arylated bismuth compound to the carrier particle via the linker species to control the rotational correlation time of the bismuth compound.The optionally substituted arylated bismuth compound may be mono-, di- or triarylated.

The synthesis of optionally substituted arylated bismuth compounds is well described in the literature Journal of Organic Chemistry, 2016, 81, 5401-5416 and references therein] and proceeds via standard organic chemistry reactions (e.g. reaction of Grignard reagents and metal halogenides) and some of them are commercially available. For example, modified bismuth compounds $Ar_2Ar'Bi$ or $Ar_2BiL$ are readily accessible via redistribution reactions starting from $Ar_3Bi$ and bismuth trihalides, selectively yielding the mono- and dihalides $Ar_2BiX$ or $ArBiX_2$. These can be reacted in a second step with e.g. metal or metalloid amides to give the respective mono- and diamides $Ar_2BiNR_2$ or $ArBi(NR_2)_2$. The mixed halides can also be reacted with nucleophiles to give the unsymmetrical organic bismuth derivatives $Ar_2BiAr'$ or $Ar_2BiL$, respectively. The diaryl bismuth amides are valuable synthons for protolytic reactions with e.g. alcohols, thiols, phenols, (carboxylic) acids etc. Hence, they provide an additional option to modify the resonance frequencies and to introduce anchor groups for crafting onto the particles or for directly attaching the bismuth centers onto the carrier particles.

In one aspect, the triarylated bismuth compound is selected from the group consisting of $Ph_3Bi$, $PhBiCl_2$, ((2,4,6-OMe)$_3$Ph)$_3$Bi, ((p-NMe$_2$)Ph)$_3$Bi and (4-fluoro-Ph)$_3$Bi, which compounds are also illustrated below.

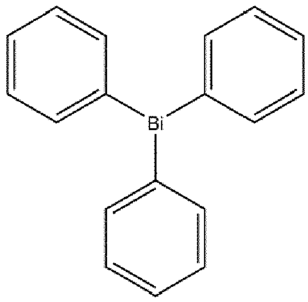

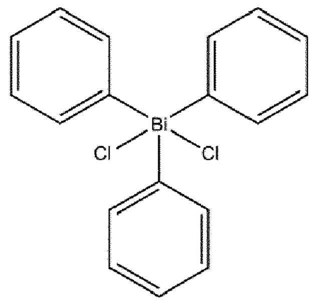

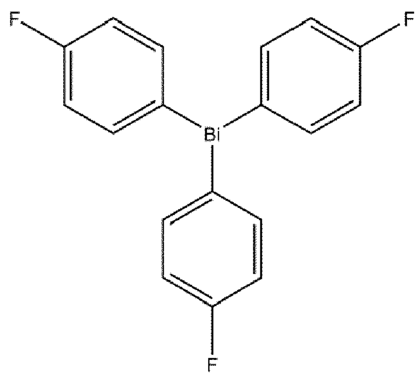

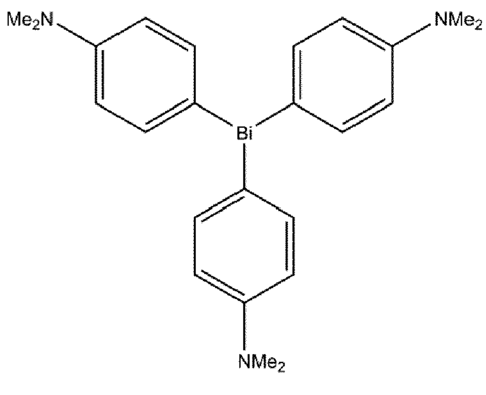

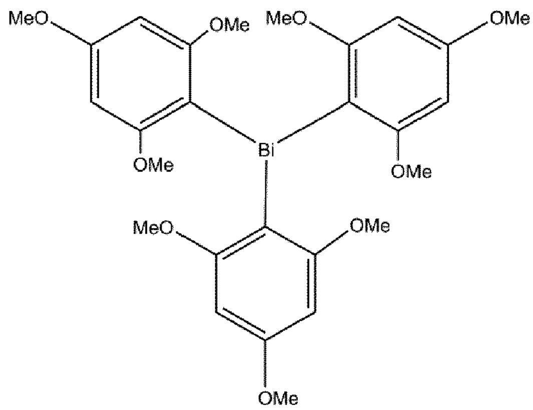

In another aspect, the arylated bismuth compound is a triarylated bismuth compound of formula (I)

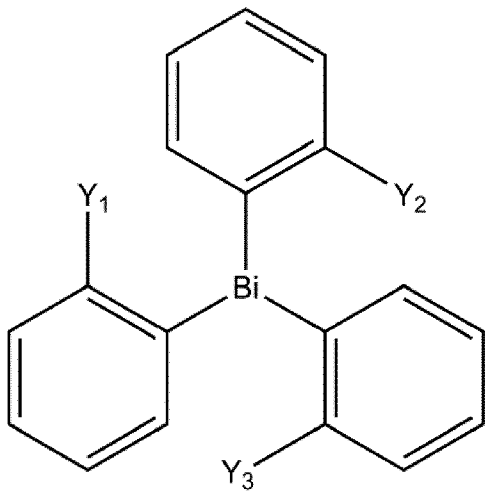

(I)

or of formula (II)

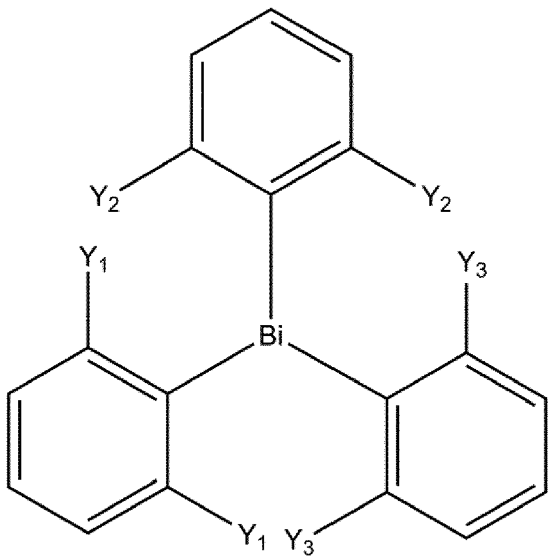

(II)

in which

$Y_1$ = $H_2C$ - $(CH_2)_n$ - Z with n = 0,1,2,3,4 or 5, and $Y_2$,$Y_3$ = H

or

$Y_1$, $Y_2$ = $H_2C$ - $(CH_2)_n$ - Z with n = 0,1, 2, 3, 4 or 5, and $Y_3$ =H

or

$Y_1$, $Y_2$, $Y_3$ = $H_2C$ - $(CH_2)_n$ - Z with n = 0,1, 2, 3,4 or 5

wherein Z = $NR^1R^2$, C1-C18 alkyl ester, C1-C18 alkyl ketone, C1-C18 alkyl aldehyde, C1-C18 alkyl ether, C1-C18 alkyl alcohol, C1-C18 alkyl thioester, C1-C18 alkyl thioketone, C1-C18 alkyl thioether, SH, C1-C18 alkyl phosphonic

acid or C1-C18 alkyl phosphite, and $R^1$ and $R^2$ are each independently selected from the group consisting of H, cycloalkyl, alkyl, alkenyl, alkynyl and aryl, preferably methyl.

The group Z in formulas (I) and (II) as well as in the other formulas (III) - (VI) below induces dative interactions with the bismuth atom, thus, allowing for a tuning of the resonance frequencies for the different transitions of the bismuth nucleus.

In still another aspect, that the arylated bismuth compound is a diarylated bismuth compound of formula (III)

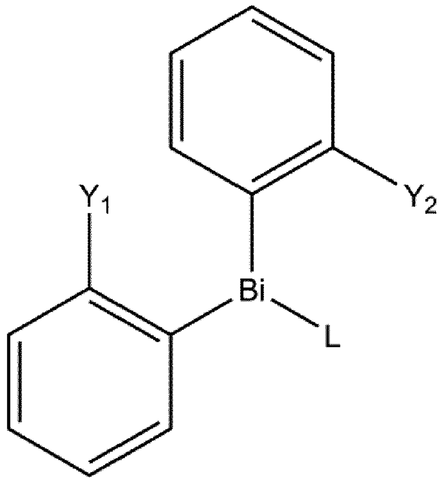

(III)

or of formula (IV)

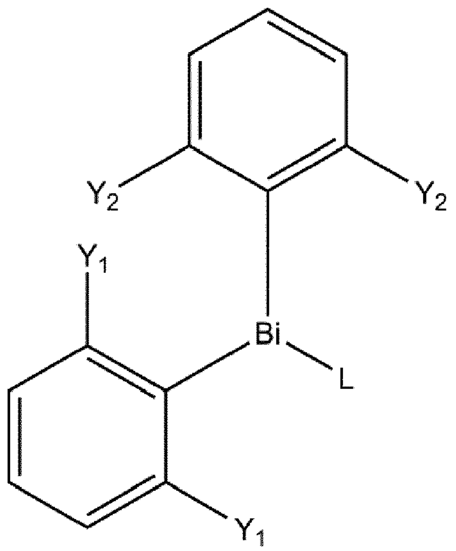

(IV)

in which

$Y_1=H_2C-(CH_2)_n-Z$ with n=0,1,2,3,4 or 5, and $Y_2=H$

or

$Y_1$, $Y_2=H_2C-(CH_2)_n-Z$ with n=0,1,2,3,4 or 5

wherein Z = $NR^1R^2$, C1-C18 alkyl ester, C1-C18 alkyl ketone, C1-C18 alkyl aldehyde, C1-C18 alkyl ether, C1-C18 alkyl alcohol, C1-C18 alkyl thioester, C1-C18 alkyl thioketone, C1-C18 alkyl thioether, SH, C1-C18 alkyl phosphonic acid or C1-C18 alkyl phosphite, and $R^1$ and $R^2$ are each independently selected from the group consisting of H, cycloalkyl, alkyl, alkenyl, alkynyl and aryl, preferably methyl;

L = aryloxy, C1-C18 alkyloxy, C5-C7 cycloalkyl, C1-C18 alkyl, C1-C18 alkenyl or tertiary C1-C18 alkyl amine.

In still another aspect, the arylated bismuth compound is a monoarylated bismuth compound of formula (V)

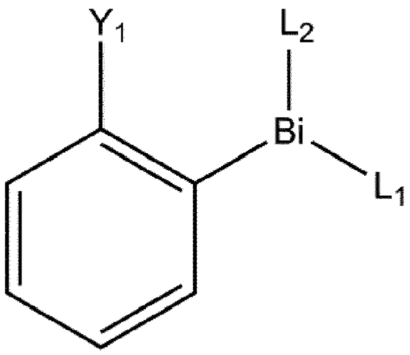

(V)

or of formula (VI)

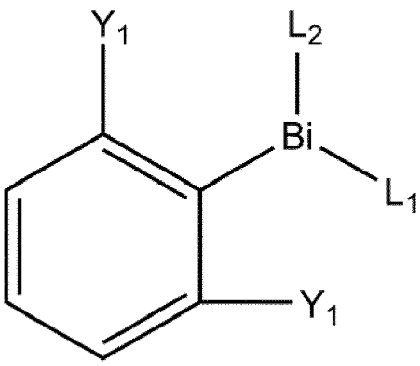

(VI)

in which

$Y_1=H_2C-(CH_2)_n-Z$ with n=0,1,2,3,4 or 5

wherein Z = $NR^1R^2$, C1-C18 alkyl ester, C1-C18 alkyl ketone, C1-C18 alkyl aldehyde, C1-C18 alkyl ether, C1-C18 alkyl alcohol, C1-C18 alkyl thioester, C1-C18 alkyl thioketone, C1-C18 alkyl thioether, SH, C1-C18 alkyl phosphonic acid or C1-C18 alkyl phosphite, and $R^1$ and $R^2$ are each independently selected from the group consisting of H, cycloalkyl, alkyl, alkenyl, alkynyl and aryl, preferably methyl;

$L_1$, $L_2$ = aryloxy, C1-C18 alkyloxy, C5-C7 cycloalkyl, C1-C18 alkyl, C1-C18 alkenyl or tertiary C1-C18 alkyl amine.

In a further aspect, n in formulas (I) - (VI) is preferably 0 or 1.

As already mentioned above, the linker (component (ii)) is preferably anchored to C4 of one of the one or more aryl group(s) of the arylated bismuth compound and allows for anchoring the optionally substituted arylated bismuth compound to the carrier particle via the linker species to control the rotational correlation time of the bismuth compound.

The attachment of chemical entities to carrier particles via linkers is well known in the art (see also description above).

The role of the carrier particle, being a nanoparticle having a size of between 5 and 200 nm, is to tune the rotational correlation time, which is connected with its size. The requirements for the nanoparticle include also to induce biocompatibility as well as to achieve sufficient stability during the measurements. The carrier particle may be a silica particle or it may be composed of a biocompatible polymer selected from the group consisting of alginates, chitosans, dextrans, chitins, celluloses, polyethylenglycols (PEG), polylactic acids (PLA), polyglycolic acid (PGA), poly-3-hydroxybutyrate (PHB), poly(lactic-co-glycolic acid) (PLGA) and polycaprolacton (PCL) and derivatives thereof. The shape of the nanoparticle can be spherical or rod like depending on the type and choice of the other components of the contrast agents.

Methods for synthesizing nanoparticles are well known in the art. The synthesis of the carrier particles/nanoparticles being composed of a biocompatible polymer may be performed via nanoprecipitation, where the polymers (or their derivatives) are dissolved in an appropriate solvent and added to a non-solvent under ultrasonication conditions. Parameters like dropping speed and concentration allow for a tuning of the nanoparticle size over several hundreds of nanometers.

In specific aspects, the contrast agent particle according to the invention may comprise a targeting species attached to the carrier particle, wherein the targeting species is preferably selected from the group consisting of (recombinant) proteins and fragments thereof (e.g. monoclonal antibodies or antibody fragments), peptides, peptidomimentic compounds, oligonucleotides (e.g. aptamers), small organic molecules, peptide nucleic acids, sugars, folate, porphyrin derivatives, and combinations thereof. Targeting species and methods for attaching these targeting species to carrier particles/nanoparticles are known to those skilled in the art.

The targeting species may be attached to the carrier particle via covalent bonding, either directly or via a linker species. Alternatively, the targeting species may be attached to the carrier particle via non-covalent bonding, e.g. via avidin-biotin-linkage. Generally, targeting species are ligands or other moieties that direct the MRI contrast agent to a specific organ or disease site. The coupling of appropriate targeting structures opens the door towards a vast number of sensing possibilities, i. e. contrasts for biomarker imaging.

The contrast agent particles according to the invention and as described herein, are particularly useful as Magnetic Resonance Imaging (MRI) contrast agents in Magnetic Resonance Imaging (MRI). As mentioned above, the contrast agent particles and MRI contrast medium of the invention allow for the exploitation of a new relaxation mechanism in MRI, the so called Quadrupole Relaxation enhancement (QRE). Accordingly, the contrast agent particles and MRI contrast medium of the invention are particularly useful in MRI, where the QRE mechanism is used.

For administration of the MRI contrast medium to subjects, human or animal, the MRI contrast medium may typically comprise a pharmaceutically acceptable carrier and/or excipient. A "pharmaceutically acceptable carrier and/or excipient" in the meaning of the present invention can be any substance used for the preparation of suitable dosage forms of MRI contrast media and known to those skilled in the art, including but not limited to coating materials, film-forming materials, fillers, disintegrating agents, release-modifying materials, carrier materials, diluents, binding agents and other adjuvants. Typical pharmaceutically acceptable excipients include substances like sucrose, manitol, sorbitol, starch and starch derivatives, lactose, and lubricating agents such as magnesium stearate, disintegrants and buffering agents.

The MRI contrast medium may be formulated for any kind of administration of contrast agents into body tissues and/or body cavities/orifices known to those skilled in the art e.g., intravenous, intramuscular, rectal, or oral administration, preferably intravenous administration.

The contrast agent particle and Magnetic Resonance Imaging contrast medium according to the invention and as described herein are particularly useful in Quadrupole Relaxation Enhancement (QRE)-based Magnetic Resonance Imaging. Accordingly, the present invention also relates to the use of these contrast agent particles and MRI contrast media in QRE-based MRI.

The MRI contrast medium according to the invention may be intended for oral administration, e.g. in form of tablets, pills, capsules or troches, but also in liquid form such as tonics, syrups, suspensions or drops. Moreover, MRI contrast medium according to the invention may be administered parenterally (e.g. intravenously, intramuscularly, intraperitoneally, subcutaneously, intracutaneously) or topically, preferably intravenously.

The present invention is further explained and illustrated by the following figures and examples.

**Fig. 1** shows a schematic view of the structure and action mechanism of the contrast agent particles according to the invention. Water molecules approach the $^{209}$Bi atom (quadrupolar nucleus QN) by translational diffusion (Dt). Protons relax with shortened $T_1$ (at specific frequencies) due to mutual dipole-dipole coupling to the QN. $k_{ex}$ symbolizes the water exchange rate, r is the distance between proton and $^{209}$Bi. The $^{209}$Bi -atom possesses ligands which, together with the external static magnetic field determine its quadrupole resonance transition frequencies. Bi and the ligands constitute the core compound, i.e. the quadrupolar compound. The low toxicity of $^{209}$Bi opens the favourable possibility to immobilise the compounds on the surface of biocompatible NPs rather than to enclose it in a chelator. This, in turn, facilitates access of water protons by translational diffusion (Dt) in the outer sphere of the bismuth compound (bulk

solvent) and direct exchange ($k_{ex}$) between the outer and the inner sphere (coordination sphere of the solvent molecules around the bismuth compound). Therefore, fewer limitations are expected for the water exchange rate than in Gd-chelates. The molecular dynamics of the spin system will be tailored by varying the size of the NPs. As explained above, the contrast agent particle according to the invention consists of 3 components: Component (i), herein also referred to as quadrupolar compound, is a comparatively small molecule with bismuth ($^{209}Bi$) as a quadrupolar nucleus in its core. This molecule is symbolized by the two phenyl rings (ligands to tune the transition frequency'). Component (ii) is the linker (symbolized as zig-zag line) and component (iii) is a nanoparticle, onto which at least one entity of component (i) is being grafted via the linker. The process of Quadrupole Relaxation Enhancement (QRE) in MRI takes place in the grey shaded zone, where the water molecules approach the bismuth. The choice of the ligands in the quadrupolar compound (component (i)) tunes the QRE frequencies to the operating frequency fo of the Magnetic Resonance (MR) scanner and is thus critical for the observation of the contrast effects at the appropriate frequency. The oscillatory ($\tau_L$) time constant of the complex formed by component (i) and linker (ii) as well as the rotational ($\tau_R$) correlation time of the whole complex determine the efficiency of QRE and hence the strength of the contrast (sensitivity). Therefore the appropriate size of the nanoparticles, being in the range of between 5 and 200 nm, is essential for a good sensitivity. This three-component structure clearly distinguishes the approach from that suggested in US 5,817,289. As far as the characteristic time constant ($\tau_L$) is concerned, the ligand oscillations contribute to fluctuations of the electric field gradient ($Vm^{-2}$) at the position of $^{209}Bi$ (i.e. the QN) and hence affect the QRE effect.

## EXAMPLES

### Example 1: Theoretical considerations and simulations for calculating the quadrupole interaction

$T_1$ relaxation of protons occurs if transitions between the energy levels associated with different magnetic quantum numbers (spin up and spin down) states are enabled so as to shift the populations of the states towards their thermal equilibrium. These transitions are induced by fluctuating magnetic fields which contain sufficient spectral power in the neighbourhood of the Larmor frequency $f_0$ given by

$$f_0 = \gamma_H B_0 \qquad (1)$$

$\gamma_H$ being the proton magnetogyric ratio (42.58 MHz/T). The source of the fluctuations for classical (paramagnetic) MRI CAs (typically $Gd^{3+}$-complexes) is rotational motion of the paramagnetic species and its coordination sphere rotating as a single body. The strong proton-electron dipole-dipole magnetic interactions produce the necessary field fluctuations at the site of the proton. The spectral distribution of the fluctuations is determined by the rotational correlation time $\tau_R$. For efficient relaxation enhancement it is important that the spectral density is high at fo and $2f_0$. Due to several restrictions this condition cannot be met in an optimal way by established $Gd^{3+}$-based CAs. Typically their efficiency is high over a broad frequency band but decreases significantly above a certain cut-off frequency.

For QRE - based CAs the dipole-dipole interactions between protons and quadrupolar nucleus (QN), which in the present invention is bismuth ($^{209}Bi$) give rise to the enhancement of the proton spin relaxation. The process becomes effective when

(1) $f_0$ comes close to one of the transition frequencies $f_{Q,k}$ of the QN: $f_0 = f_{Q,k}$
(2) The rotational correlation time is long enough to fulfill the condition $2\Pi f_{Q,k\tau R} > 1$

Fluctuations of the EFG also play an important role in QRE effects. The fluctuations are responsible for the QN relaxation,$T_{1Q}$ and $T_{2Q}$. Their characteristic time constant (correlation time) $\tau_{EFG}$ is determined by different sources of internal dynamics of the compound. The quadrupolar relaxation is usually much faster than the proton relaxation and thus it can efficiently drive the QN to its thermal equilibrium.

There is no simple mathematical equation which describes the QRE. To describe the enhancement of proton relaxation caused by the interactions with QNs one needs complex computations based on the stochastic Liouville formalism. Tailoring the right components is extremely facilitated by such quantum-physical simulations. As the simulations rely on an extremely sophisticated procedure with many tuning parameters, it is wise to split them into at least two sub-tasks:

(1) simulation of the QN spin transitions which determine the QRE frequencies by varying the two parameters, namely Qcc and $\eta$ - to tune them to the correct frequency range.

(2) simulation of the QRE pattern by means of the stochastic Liouville formalism involving the Qcc and $\eta$ values

defined in step (1). By varying the rotational correlation time ($\tau_R$) and the characteristic time constant of EFG fluctuations ($\tau_{EFG}$) one can estimate the size of the carrier particles needed to detect QRE. For spherical particles the rotational correlation time $\tau_R$ is linked to the hydrodynamic radius r by the relationship

$$\tau_R = \frac{4\pi\eta r^3}{3kT} \tag{3}$$

whereby $\eta$ denotes viscosity (not the asymmetry parameter!), k Boltzmann's constant and T the absolute temperature.

The energy level structure of the quadrupolar nucleus (QN) can be found by diagonalizing the total Hamiltonian containing the quadrupole interaction (represented in the reference frame determined by the external magnetic field) and the Zeeman interaction of the QN.

$$H = H_Q + H_Z$$

$$H_{Q,PAS} = \frac{e^2qQ}{4I(2I-1)}\left(3\hat{I}_z^2 - \hat{I}^2 + \eta\frac{\hat{I}_x^2 - \hat{I}_y^2}{2}\right)$$

$$H_Z = \gamma_Q h B_0$$

whereas $H_Q$ is the Hamiltonian of the pure quadrupole interaction in the Zeeman system and can be calculated by transforming the principal axis form $H_{Q,PAS}$ into the Zeeman system via the Wigner rotation matrices. $H_z$ is the pure Zeeman Hamiltonian which depends on the static magnetic flux density Bo.

I is the spin quantum number I of the QN. $e^2qQ$ is the largest principal component of the tensor product between the quadrupole moment and the EFG. $\eta$ is a measure for the deviation of the EFG tensor from axial symmetry and can vary between 0 (ideally axially symmetric) and 1 (extremely asymmetric). The transition probabilities can be calculated as usually from the spin matrices $I_x$ und $I_y$ after transformation into the eigensystem of the total Hamiltonian.

Fig. 2 shows the calculated transitions between the energy levels of $BiPh_3$, a quadrupolar compound as it is used in contrast agent particles of the present invention, as a function of the magnetic field Bo in the range of 0 - 3.5T (solid curves). The data was simulated for pure mono-crystals assuming that principal axis of the EFG and the Zeeman (Bo) axis coincide. The expected QRE should occur at magnetic fields close to the crossings of the transition frequencies and the line representing the proton Larmor frequency (dashdotted straight line).

The uppermost crossing at 134.5 MHz occurs already close to the proton Larmor frequency at 3T, namely ca. 123.4 MHz. For this reason $BiPh_3$ is considered as a promising starting compound. The transition frequencies can be tuned by either adding ligands to one or more of the phenyl rings close to the $^{209}Bi$ atom or substituting the whole rings by other ligands ($Ar_3Bi \rightarrow Ar_2BiL \rightarrow ArBiL_2$). To lower the uppermost transition frequency it is e. g. necessary to lower the EFG inhomogeneity i.e. by lowering the asymmetry of the molecule. In contrast also lower transitions can be shifted upwards by increasing the asymmetry. Furthermore, it is possible to increase the coordination number around bismuth from three in $Ar_3Bi$ to higher values by introducing chelating side arms attached to e.g. the aryl substituents.

By comparing the published NQR data, Bi transitions in a majority of $^{209}Bi$-compounds are between 40 and 120 MHz, making $^{209}Bi$ a very favorable candidate for contrast agents operating at 3T and 1.5T, respectively.

*Table* 1 summarizes the abundance, NMR and NQR parameters for $^{209}Bi$. Q is the quadrupole moment:

| | Natural Abundance % | Spin I | $\gamma_Q$ (MHz/T) | Q ($10^{-24}$ $cm^2$) |
|---|---|---|---|---|
| $^{209}Bi$ | 100 | 9/2 | 6.84 | -0.4 |

**Example 2: Chemical structure and NQR data for selected quadrupolar bismuth compounds tested in NQR spectroscopy.**

Example 2 describes the following quadrupolar bismuth compounds to be used as components for the contrast agent particles and MRI contrast media, respectively, according to the invention, namely $Ph_3Bi$, $Ph_3BiCl_2$, $((2,4,6\text{-}OMe)_3Ph)_3Bi$, $((p\text{-}NMe_2)Ph)_3Bi$ and $(4\text{-fluoro-}Ph)_3Bi$:

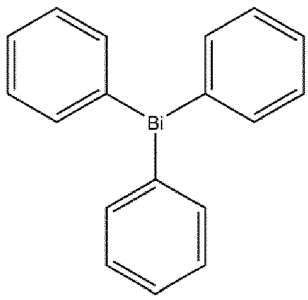

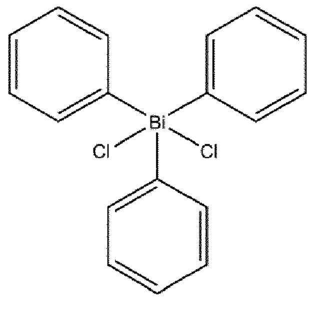

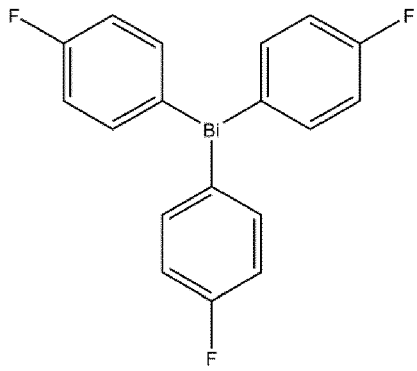

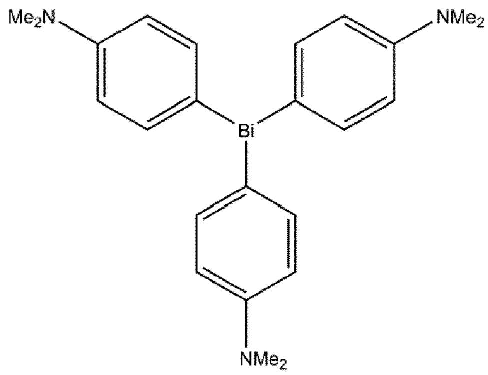

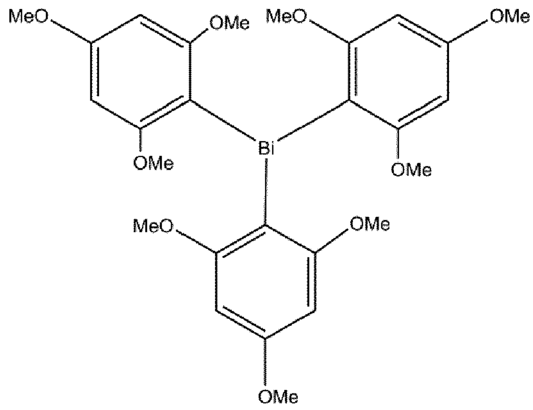

The core structure of the bismuth compounds is triphenyl bismuth ($Ph_3Bi$). In order to match the required resonance frequency for clinical scanners and tailor the water exchange rates , the polarity, basicity and hydrophilicity of the aryl rings can be altered at C2. In order to achieve a contrast agent particle according to the invention, these bismuth compounds are then attached by means of a linker via C4 of the aryl ring(s) to a carrier in order to tune the rotational correlation time. These compounds showed Bi transition frequencies close to the desired ones. In table 2 an overview of those compounds and the NQR parameters which have been determined so far are given. In the last column the most important number is $f_{close,2.89T}$, which denotes the NQR transition frequency calculated for 2.89T, being closest to the proton Larmor frequency $f_0$ of a clinical SIEMENS Skyra MRI scanner. (Remark: The Skyra operates at 2.89T instead of true 3T, which corresponds to an fo of 123.4MHz). It should be emphasized, however, that due to the solvent exchange processes and the quadrupole spin relaxation the QRE frequency can somewhat deviate from $f_{close,2.89T}$.

As can be seen, there are some distinct differences between the different compounds. The NQR transition frequencies depend strongly on the oxidation state of Bi as can be seen in table 2. When the oxidation state of bismuth is increased from +3 to +5 a concomitant increasing of Qcc from about 660 to ca. 1039 MHz ($BiPh_3Cl_2$) is observed. Therefore $f_{close,2.89T}$ corresponds to the transition $7/2 \rightarrow 5/2$ rather than $9/2 \rightarrow 7/2$ as for all the other compounds. As already mentioned the NQR transition frequencies can also be tuned by the attached ligands. Indeed, smaller but still significant changes are obtained when the environment of the Bi nucleus is changed, for instance by attaching trimethoxy groups in positions 2 and 6. Additionally, chelating side arms attached to the aromatic rings strongly influences the NQR transitions. However, the effects caused by the structural alterations are much smaller than those related to the oxidation state and this is a clear indication that fine tuning of the frequency in small steps is possible. Different substituents in para position have also been tested to explore their impact on the transition frequencies. As shown in table 2, the attachment of groups has a slight influence on $f_{close,2.89T}$, the best match with the target frequency (only 6.5 MHz difference) being obtained with $Bi(p\text{-}F\text{-}Ph)_3$.

*Table 2:* Transition frequencies $f_n$ and quadrupolar parameters (Qcc and $\eta$) of selected bismuth compounds at different temperatures; $f_1$ denotes the 3/2->1/2, $f_2$ the 5/2->3/2, $f_3$ the 7/2->5/2 and $f_4$ the 9/2->7/2 transition at zero magnetic field. T denotes temperature, RT room temperature (25°C). Compound 1 and 5 have also been characterized at 37°C. $f_{CO,2.89T}$ in the last column represents the NQR transition frequency (transition indicated in brackets) which lies closest to the proton Larmor frequency $f_0$ of a clinical SIEMENS Skyra MRI scanner (officially 3 T, effectively 2.89T), i. e. 123.4 MHz. n.m. means 'not yet measured'.

| | T [°C] | $f_1$ [MHz] | $f_2$ [MHz] | $f_3$ [MHz] | $f_4$ [MHz] | Qcc [MHz] | η [1] | $f_{close,2.89T}$ |
|---|---|---|---|---|---|---|---|---|
| $BiPh_3$ | RT | 29.755 | 55.300 | 83.500 | 111.400 | 668.71 | 0.075 | 131.7 (f4) |
| | 37 | n.m. | n.m. | 83.423 | 111.319 | 668.31 | 0.087 | 131.6 (f4) |
| | | | | | | | | |
| $Bi((2,4,6\text{-}MeO)_3Ph)_3$ | RT | n.m. | 56.940 | 85.550 | 113.870 | 679.68 | - | 133.6 (f4) |
| $Bi(p\text{-}F\text{-}Ph)_3$ | RT | n.m. | 54.500 | 82.200 | 109.600 | 657.60 | - | 129.9 (f4) |
| $Bi(p\text{-}NMe_2Ph)_3$ | RT | n.m. | n.m. | 82.580 | 110.040 | 659.04 | - | 130.4 (f4) |
| $BiPh_3Cl_2$ | RT | n.m. | n.m. | 86.471 | n.m. | n.m. | n.m. | |
| | 37 | n.m. | n.m. | 86.401 | 129.910 | 1039.7 | 0.046 | 109.6 (f3) |

It is concluded that the oxidation state has the largest influence on transition frequencies for the triarylsubstituted bismuth compound systems, followed by substitution on C2 of the adjacent aryl (phenyl) rings and to a somewhat minor extent substitution in the para position at C4. Since the investigated compounds have already promising frequencies close to the desired targets, the fine tuning using substitution at C2 is preferred, whereas substitution at C4 allows for anchoring a nanoparticle, i.e. carrier particle having a particle size of between 5 and 200 nm, via a linker to control the rotational correlation time of the bismuth containing compound.

**Example 3: Potential of the invention: theoretical expectations**

The proton spin-lattice relaxation rate $R_1 = 1/T_1$ caused by proton-Bi dipole-dipole interactions was simulated with the stochastic Liouville theory [see e.g. Kruk et al, Solid State Nucl. Magn. Reson., Vol. 40, No. 3, p. 114-120, Okt. 2011 and "Understanding Spin Dynamics" in CRC Press, 16 Oct 2015, ISBN 9789814463492.]. Fig. 3 shows simulated $R^1$ dependences on the magnetic field for protons rigidly attached to the $^{209}$Bi nucleus for different rotational correlation times $\tau_R$. One can clearly see the strong narrowing of the peak and the increase of its amplitude at specific frequencies for longer rotational correlation times. The $\tau_R$ values approximately correspond to rotation of a rigid sphere of the radius of ca. 5-15 nm in blood. Fig.4 shows a ratio between the relaxation rate $R^1$ caused by $^1$H-$^{209}$Bi dipole-dipole interactions and the $R_{1HH}$ relaxation rate originating from proton-proton dipolar interactions for the same rotational correlation time and inter-spin distance. From this figure one can estimate the expected order of the relaxation enhancement depending on the time-scale of the rotational dynamics.

However, there are more parameters which strongly influence the QRE peaks. An important factor is the correlation time $\tau_{EFG}$ and the amplitude of the fluctuations of the EFG. Fig. 5 shows QRE peaks affected by fluctuations of the electric field gradient tensor ("ratio" describes the ratio between the amplitude of the fluctuations of the quadrupole interactions and its main (averaged) value). As can be seen the peak gets broader and less pronounced when the EFG fluctuations increase in amplitude. As $\tau_{EFG}$ and the amplitude of EFG fluctuations determine the quadrupolar relaxation rates, zero field NQR spectroscopy providing information on the quadrupole relaxation is an important method for characterizing and pre-selecting the synthetized molecules. The asymmetry parameter has a strong influence on the positions of the quadrupole peaks. **Fig. 6** shows how the transition frequencies $f_{Q,k}$ depend on $\eta$.

Finally, when designing the systems it is important to realize that water molecules should have the opportunity to closely approach the 209Bi nucleus. Otherwise the QRE between the water protons and the quadrupolar nucleus will be dominated by $^1$H-$^1$H relaxation contributions involving protons belonging to the quadrupolar compound.

**Example 4: Synthesis of a contrast agent particle according to the invention**

**Synthesis of $Bi(C_6H_5)_3$ (1):** A solution of PhMgBr (prepared from PhBr (44.81g, 285,.4 mmol) and Mg (8.32g, 343.0 mmol) in diethyl ether (100 ml)) in diethyl ether is added over 1 h to a suspension of $BiCl_3$ (25.0g, 79 mmol) in diethyl ether which was cooled to 0°C. The reaction mixture was heated to room temperature and stirred for 24 h. Afterwards it was quenched and washed with water. Furthermore, the raw product was recrystallized from hot ethanol. Triphenylbismuth **(1)** was isolated as colourless crystals. Yield 28.0 g (80%). mp 77-78 °C; $\delta_H$ (300 MHz, $CDCl_3$, ppm) 7.31-7.42 (9H, m, CH), 7.75 - 7.77 (6H, d, CH); $\delta_C$ (75 MHz, $CDCl_3$, ppm) 127.89 (CH), 130.64 (CH), 137.68 (CH).

**Synthesis of $Bi(C_6H_5)_2Cl$ (2):** $BiCl_3$ (7.16 g, 22.7 mmol) was added in small portions to a solution of **(1)** (10.0 g, 22.7 mmol) in 150 ml diethyl ether at 0 °C. After 1 h the solvent was removed and the colourless precipitate was washed three times with 100 ml of diethyl ether and dried under reduced pressure. Yield 8.95 g (99%). mp 185 °C; $\delta_H$ (300 MHz, DMSO-$d_6$, ppm) 7.25 - 7.30 (2H, t, CH), 7.53 - 7.58 (4H, t, CH), 8.24 - 8.27 (4H, d, CH); $\delta_C$ (75 MHz, DMSO-$d_6$, ppm) 127.66 (CH), 131.21 (CH), 137.06 (CH).

**Synthesis of $Bi(C_6H_5)_2N(CH(CH_3)_2)_2$ (3):** $LiN(CH(CH_3)_2)_2$ (1.36 g, 12.50 mmol) in 60 ml THF was added to a suspension of **(2)** (5.00 g, 12.50 mmol) in THF (60 ml). Both mixtures were previously cooled to -78 °C. The solution was stirred for 3 h slowly warming to room temperature and evaporated to dryness. The residue was dissolved in 60 ml of *n*-pentane and filtered through Kieselgur and the resulting solution was evaporated to dryness under vacuum. A yellow product was obtained. Yield 4.39 g (76%). mp 35.0 °C; $\delta_H$ (300 MHz, $C_6D_6$, ppm) 1.01 - 1.03 (12H, d, $CH_3$), 3.98 - 4.11 (2H, sept, CH) 7.10 - 7.15 (2H, t, CH), 7.26 - 7.31 (4H, t, CH), 7.85 - 7.88 (4H, d, CH); $\delta_C$ (75 MHz, $C_6D_6$, ppm) 28.46 ($CH_3$), 52.34 (CH), 128.26 (CH), 130.65 (CH), 137.28 (CH).

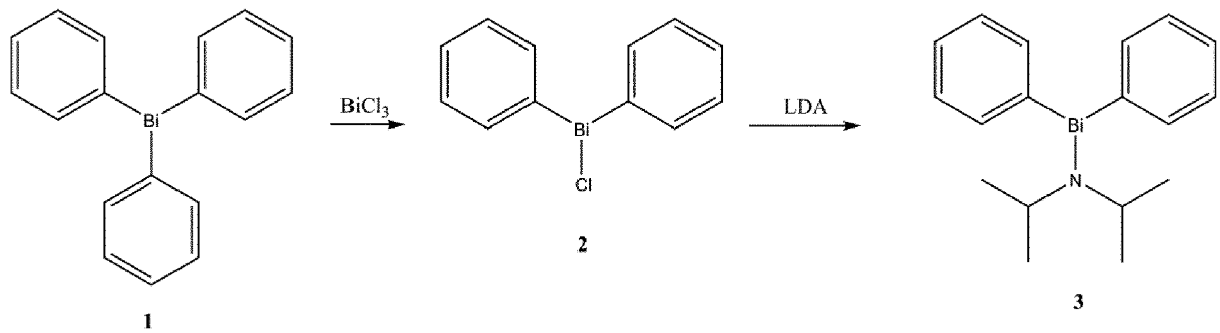


1

2

3

**Synthesis of $Bi(C_6H_5)_2N(Si(CH_3)_3)_2$ (4):** $LiN(Si(CH_3)_3)_2$ (2.52 g, 15.05 mmol) in 60 ml THF was added to a

suspension of (2) (6.00 g, 15.05 mmol) in THF (60 ml). Both mixtures were previously cooled to -78 °C. The solution was stirred for 3 h slowly warming to room temperature and evaporated to dryness. The residue was dissolved in 60 ml of *n*-pentane and filtered through kieselguhr and the resulting solution was evaporated to dryness under vacuum. A slightly yellow solid was obtained. Yield 7.10 g (90 %). mp 72.9 °C; $\delta_H$ (300 MHz, $C_6D_6$, ppm) 0.22 (18H, s, $CH_3$), 7.09 - 7.14 (2H, t, CH) 7.32 - 7.37 (4H, t, CH), 7.94 - 7.96 (4H, d, CH); $\delta_C$ (75 MHz, $C_6D_6$, ppm) 6.78 ($CH_3$), 128.58 (CH), 131.01 (CH), 137.15 (CH); $\delta_{Si}$ (60 MHz, $C_6D_6$, ppm) 6.37.

**Synthesis of 4-(Prop-2-yn-1-yl)phenol (5):** Hydroquinone (11.0 g, 0.910 mmol) and propargyl chloride (7.50 g, 0.101 mmol) were dissolved in ethanol (20 mL) under an argon atmosphere in a three-neck flask equipped with an addition funnel. The reaction was heated to reflux to dissolve all solids. KOH (0.10 M in water) was added drop wise through the addition funnel. The mixture was then stirred for 24 h under cooling. After removal of the volatiles in vacuo, $CH_2Cl_2$ was added and dilute, aqueous KOH was added. After isolation of the aqueous layer, and neutralization using 1 M HCl then brought to a neutral pH by the addition of 1 M HCl and extracted with $CH_2Cl_2$. The combined organic layers were further washed with water, dried, filtered and concentrated. Yield 2.2 g, 17%. $^1H$ NMR $\delta_H$ (300 MHz, $CDCl_3$, ppm) (ppm) 6.85 (dd, J = 9.1,1.1 Hz, 2H), 6.78 (d, J = 9.0 Hz, 2H), 4.61 (dd, J = 2.4,1.0 Hz, 2H), 2.50 (t, J = 2.4 Hz, 1H).

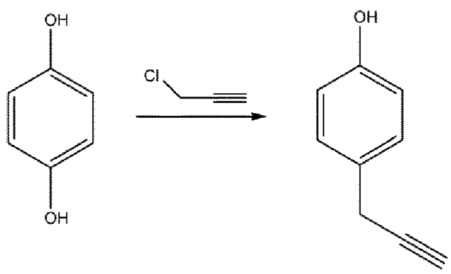

**Synthesis of [4-(Prop-2-yn-1-yl)]phenoxy-Bi($C_6H_5$)$_2$ (6):** 1.0 g of **(3)** (2.2 mmol) are dissolved in pentane (20 ml) and **(5)** (0.39 g, 2.9 mmol) are added via spatula at 0°C under vigorous stirring. After one hour of stirring, the mixture is heated to 50°C for a period of one hour. After cooling to room temperature, the volatiles are removed in vacuo and washed twice with water. After drying, 0.95 g of **(6)** were isolated (88% yield.)

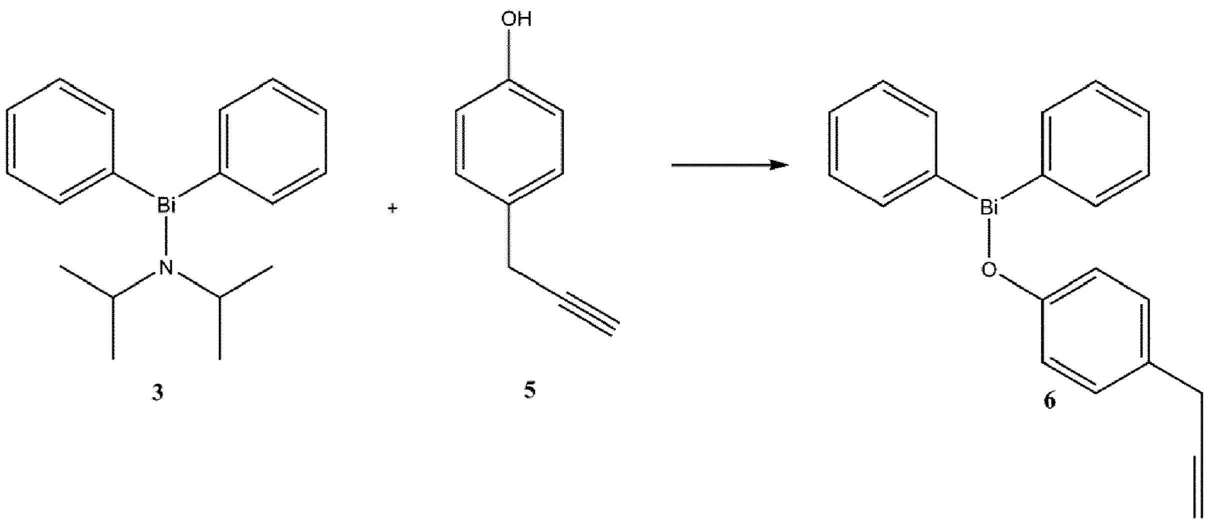

**Synthesis of 6-deoxy-6-azido cellulose (7):** 2.5 g (0.01543 mol) cellulose and 100 ml dry DMA are stirred for 3 h at 110 °C and afterwards 5.5 g LiCl are added. After stirring overnight at room temperature, a solution of 7.3 ml (0.05 mol, 3.40 mol/mol AGU) triethylamine in 7.3 ml DMA is added dropwise within one hour followed by slow addition of 5.0 g (0.03 mol, 1.70 mol/mol AGU) tosylchloride in 10 ml DMA. Then, the mixture is stirred for 6 h at room temperature before 5.0 g (0.08 mol) $NaN_3$ are added. The mixture is allowed to stir for 16 hours at 100°C. After cooling down to room temperature, the reaction mixture is poured into 500 ml iced water. The precipitate is filtered off, washed thoroughly with water, ethanol and it is dried in vacuum at 50° C. Yield: 2.0 g.

**Reaction of 6-deoxy-6-azido cellulose (7) with [4-(Prop-2-yn-1-yl)]phenoxy-Bi($C_6H_5$)$_2$ (6)**

(7) (0.3 g, 1.64 mmol) was dissolved in DMSO (30 ml) and reacted with **(6)** (0.84 g, 1.6 mmol) in the presence of copper(II) sulfate pentahydrate (0.01 g, 0.05 mmol) and sodium ascorbate (0.02 g, 0.1 mmol). After stirring the mixture at 70° C for 24 h it was precipitated in 75 ml of methanol and the polymer was collected by filtration and washed with methanol and finally dried in vacuo to yield 0.9 g (79%) of **(8).**

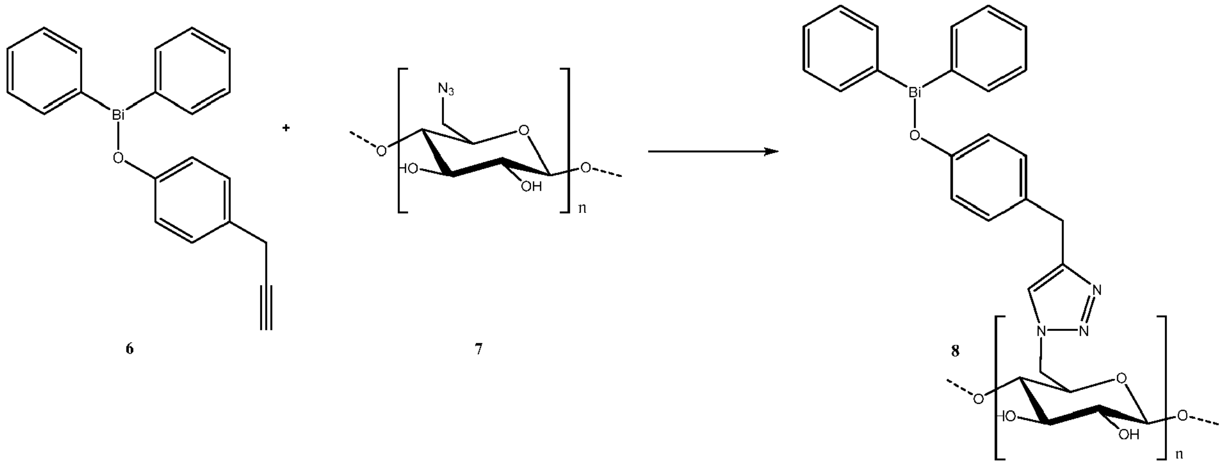

6 7 8

**Synthesis of nanoparticles by nanoprecipitation:** In a typical procedure, **(8), (10)** and **(12)** (0.05-5.0 g/l) was dissolved in N,N-dimethylacetamide and precipitated in a non-solvent (e.g. water) under sonication by slowly dropping the solution into the water via a dropping funnel. Depending on the used concentration of the solution, particles with a diameter in a range from 20 (0.05 g/L) to 400 nm (5.0 g/L) are obtained. After separation of the nanoparticles by centrifugation, the particles are washed with the non-solvent and lyophilized to give white powders.

**Synthesis of (4-ethinylphenyl)diphenyl bismuthine, (9)**

N-butyl lithium (1.6 ml solution in THF, 5.2 mmol) was added via syringe to an anhydrous THF solution (50 ml) of (4-bromophenyl)ethinyltrimethylsilane (1.3 g, 5 mmol) and allowed to stir for 2 h at -78°C. Bi($C_6H_5$)$_2$Cl (2) (2 g, 5 mmol) dispersed in 35 ml anhydrous THF was added to the reaction mixture, and stirred at -78°C for 2 h. The reaction was allowed to warm to room temperature overnight and tetrabutylammonium fluoride (TBAF, 5.2 ml in THF solution 1.0 M, 5.2 mmol) was added to the reaction mixture. After 4 h of stirring at room temperature, the solvent was removed in vacuo, and the residue was purified by column chromatography whereas a colorless oil was obtained (1.5 g, 65%)

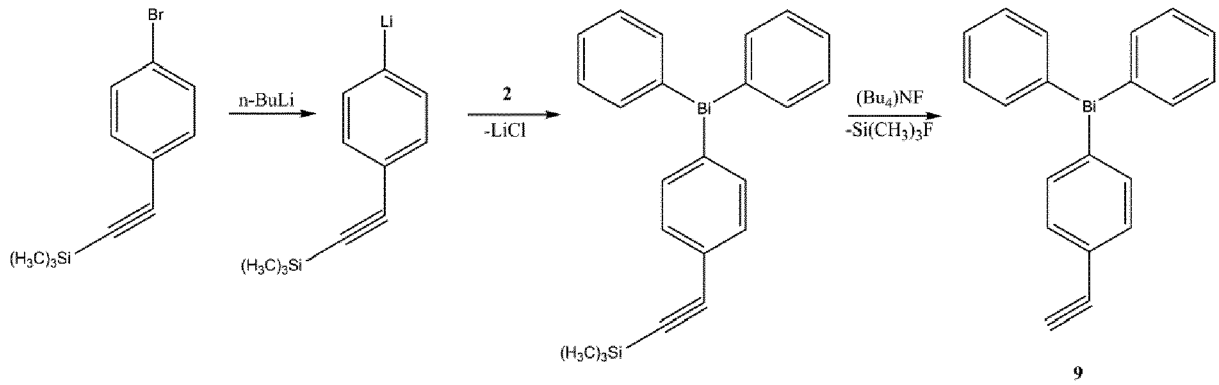


9

**Reaction of 6-deoxy-6-azido cellulose (7) with (4-ethinylphenyl)diphenyl bismuthine (9)**

(7) (0.3 g, 1.64 mmol) was dissolved in DMSO (30 ml) and reacted with **(9)** (0.75 g, 1.6 mmol) in the presence of copper(II) sulfate pentahydrate (0.01 g, 0.05 mmol) and sodium ascorbate (0.02 g, 0.1 mmol). After stirring the mixture at 70° C for 24 h it was precipitated in 75 ml of methanol and the polymer was collected by filtration and washed with methanol and finally dried in vacuo to yield 0.8 g (70%) of **(10).**

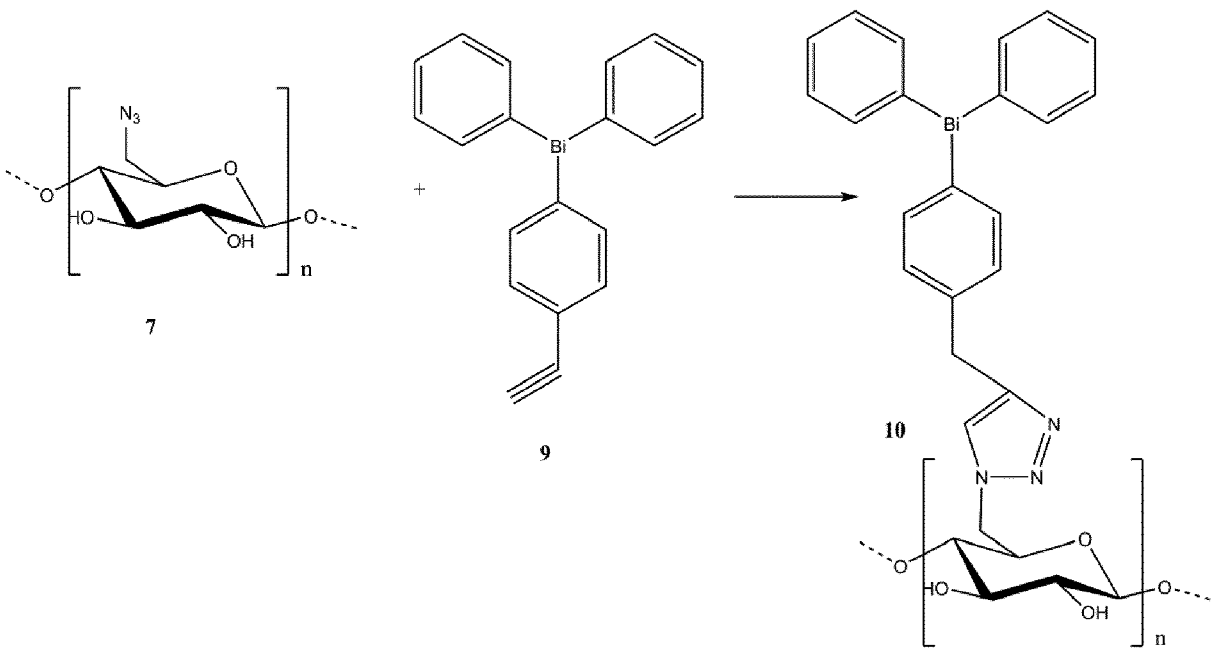

7

9

10

**Synthesis of (4-ethinylphenyl)diphenoxy bismuthine, (11)**

N-butyl lithium (1.6 ml solution in THF, 5.2 mmol) was added via syringe to an anhydrous THF solution (50 ml) of (4-bromophenyl)ethinyltrimethylsilane (1.3 g, 5 mmol) and allowed to stir for 2 h at -78°C. Diphenoxybismuth chloride (2.2 g, 5 mmol) dissolved in 35 ml anhydrous THF was added to the reaction mixture, and stirred at -78°C for 2 h. The reaction was allowed to warm to room temperature overnight and tetrabutylammonium fluoride (TBAF, 5.2 ml in THF solution 1.0 M, 5.2 mmol) was added to the reaction mixture. After 4 h of stirring at room temperature, the solvent was removed in vacuo, and the residue was purified by column chromatography whereas a white-yellowish solid was obtained (0.2 g, 8%)

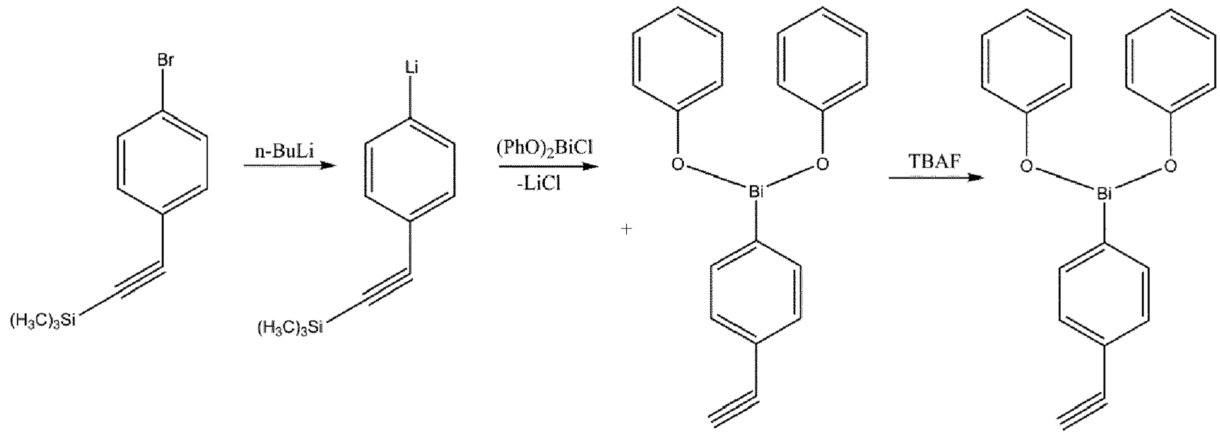


11

**Reaction of 6-deoxy-6-azido cellulose (7) with (4-ethinylphenyl)diphenoxy bismuthine (11)**

**(7)** (0.3 g, 1.64 mmol) was dissolved in DMSO (30 ml) and reacted with **(11)** (0.79 g, 1.6 mmol) in the presence of copper(II) sulfate pentahydrate (0.01 g, 0.05 mmol) and sodium ascorbate (0.02 g, 0.1 mmol). After stirring the mixture at 70° C for 24 h it was precipitated in 75 ml of methanol and the polymer was collected by filtration and washed with methanol and finally dried in vacuo to yield 0.8 g (67%) **of (12).**

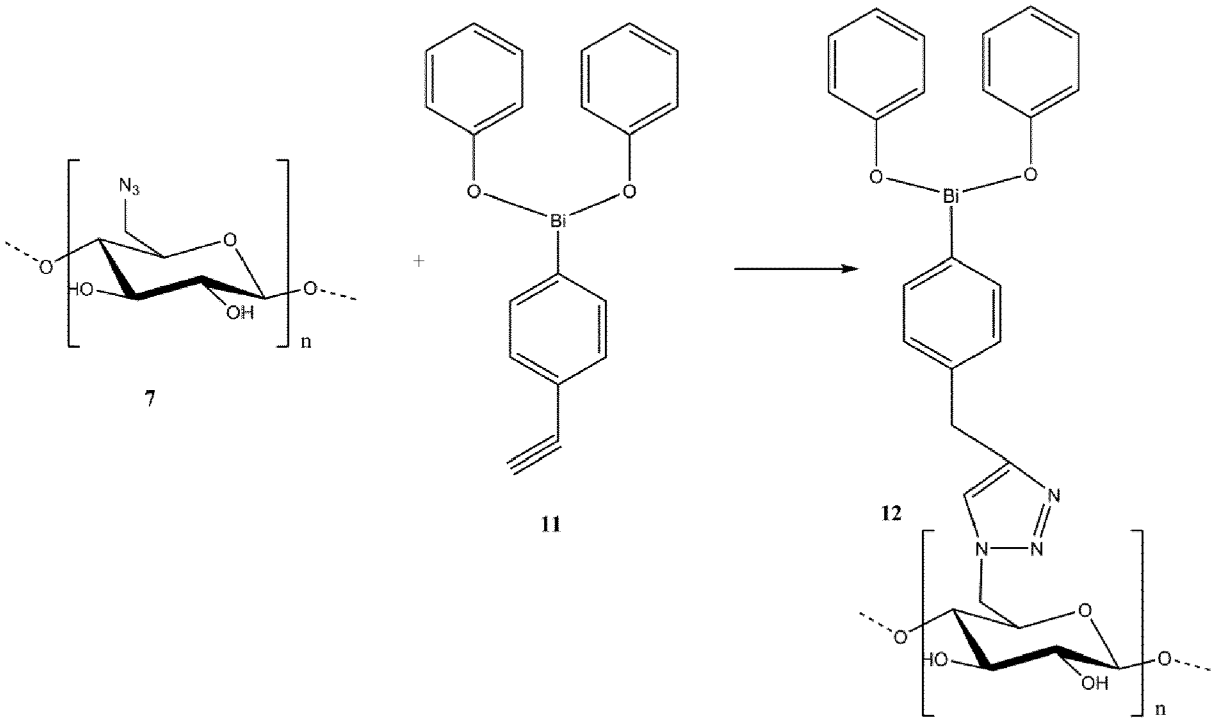

7

11

12

## Claims

1. A contrast agent particle comprising a carrier particle having a particle size of between 5 and 200 nm and at least one quadrupolar compound comprising at least one bismuth as a quadrupolar nucleus, wherein the at least one quadrupolar compound is bound to the carrier particle via at least one linker.

2. The contrast agent particle of claim 1, **characterized in that** the at least one quadrupolar compound is covalently bound to the carrier particle via the at least one linker.

3. The contrast agent particle of claim 1 or 2, **characterized in that** the at least one quadrupolar compound is an optionally substituted arylated bismuth compound.

4. The contrast agent particle of claim 3, **characterized in that** the optionally substituted arylated bismuth compound is mono-, di- or triarylated.

5. The contrast agent particle of claim 4, **characterized in that** the arylated bismuth compound is a triarylated bismuth compound selected from the group consisting of $Ph_3Bi$, $Ph_3BiCl_2$, $((2,4,6\text{-}OMe)_3Ph)_3Bi$, $((p\text{-}NMe_2)Ph)_3Bi$ and $(4\text{-fluoro-}Ph)_3Bi$.

6. The contrast agent particle of claim 4, **characterized in that** the arylated bismuth compound is a triarylated bismuth compound of formula (I)

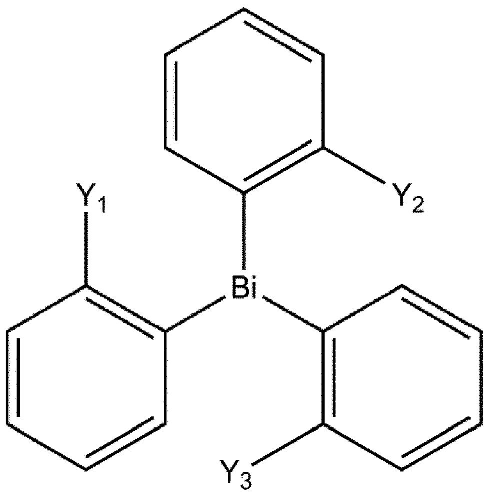

(I)

or of formula (II)

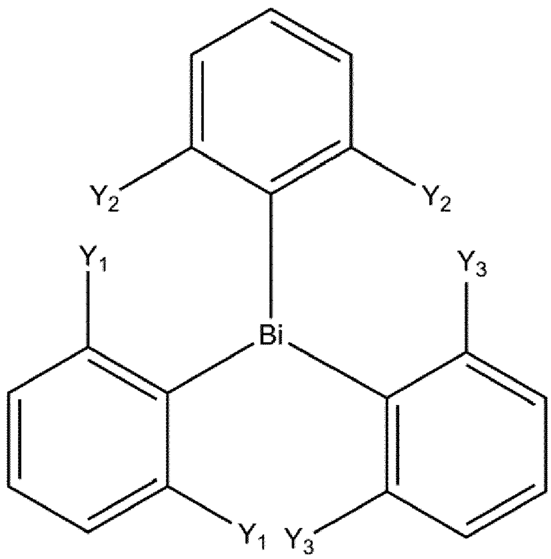

(II)

in which

$Y_1 = H_2C - (CH_2)_n - Z$ with n = 0,1,2,3,4 or 5, and $Y_2, Y_3 = H$
or
$Y_1, Y_2 = H_2C - (CH_2)_n - Z$ with n = 0,1, 2, 3, 4 or 5, and $Y_3 = H$
or
$Y_1, Y_2, Y_3 = H_2C - (CH_2)_n - Z$ with n = 0,1, 2, 3,4 or 5
wherein $Z = NR^1R^2$, C1-C18 alkyl ester, C1-C18 alkyl ketone, C1-C18 alkyl aldehyde, C1-C18 alkyl ether, C1-C18 alkyl alcohol, C1-C18 alkyl thioester, C1-C18 alkyl thioketone, C1-C18 alkyl thioether, SH, C1-C18 alkyl phosphonic acid or C1-C18 alkyl phosphite, and $R^1$ and $R^2$ are each independently selected from the group consisting of H, cycloalkyl, alkyl, alkenyl, alkynyl and aryl, preferably methyl.

**7.** The contrast agent particle of claim 4, **characterized in that** the arylated bismuth compound is a diarylated bismuth compound of formula (III)

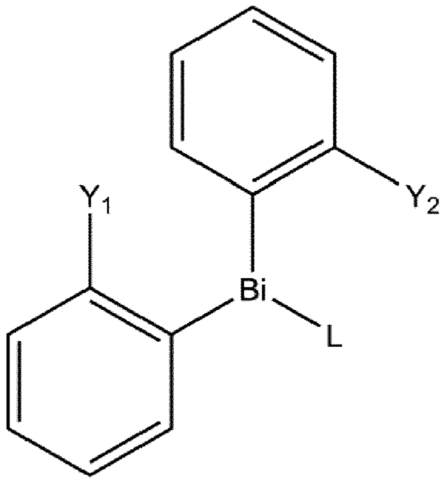

(III)

or of formula (IV)

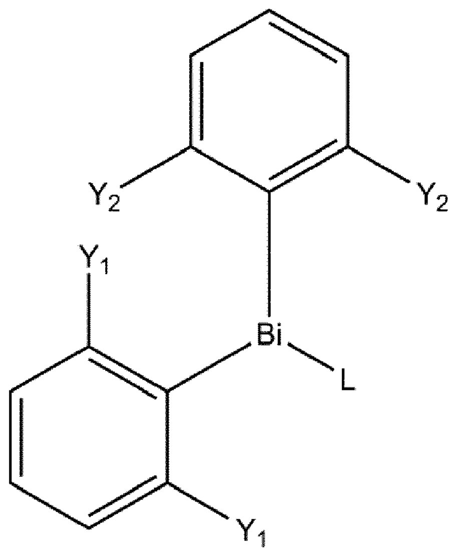

(IV)

in which

$Y_1$=$H_2C$-$(CH_2)_n$ -Z with n=0,1,2,3,4 or 5, and $Y_2$=H
or
$Y_1$,$Y_2$=$H_2C$-$(CH_2)_n$ -Z with n=0,1,2,3,4 or 5
wherein Z = $NR^1R^2$, C1-C18 alkyl ester, C1-C18 alkyl ketone, C1-C18 alkyl aldehyde, C1-C18 alkyl ether, C1-C18 alkyl alcohol, C1-C18 alkyl thioester, C1-C18 alkyl thioketone, C1-C18 alkyl thioether, SH, C1-C18 alkyl phosphonic acid or C1-C18 alkyl phosphite, and $R^1$ and $R^2$ are each independently selected from the group consisting of H, cycloalkyl, alkyl, alkenyl, alkynyl and aryl, preferably methyl;
L = aryloxy, C1-C18 alkyloxy, C5-C7 cycloalkyl, C1-C18 alkyl, C1-C18 alkenyl or tertiary C1-C18 alkyl amine.

8. The contrast agent particle of claim 4, **characterized in that** the arylated bismuth compound is a monoarylated bismuth compound of formula (V)

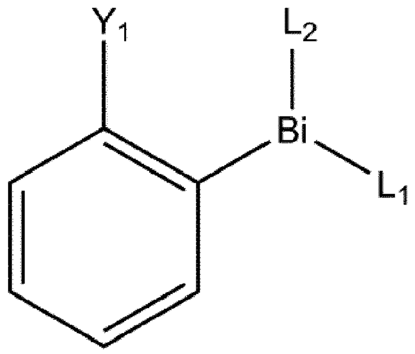

(V)

or of formula (VI)

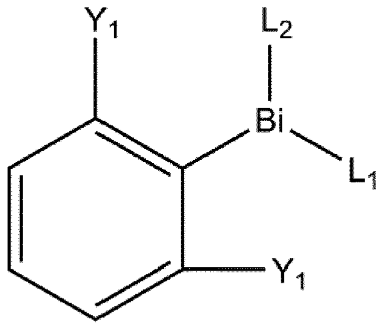

(VI)

in which

$Y_1=H_2C-(CH_2)_n$ -Z with n=0,1,2,3,4 or 5
wherein Z = $NR^1R^2$, C1-C18 alkyl ester, C1-C18 alkyl ketone, C1-C18 alkyl aldehyde, C1-C18 alkyl ether, C1-C18 alkyl alcohol, C1-C18 alkyl thioester, C1-C18 alkyl thioketone, C1-C18 alkyl thioether, SH, C1-C18 alkyl phosphonic acid or C1-C18 alkyl phosphite, and $R^1$ and $R^2$ are each independently selected from the group consisting of H, cycloalkyl, alkyl, alkenyl, alkynyl and aryl, preferably methyl;
$L_1$, $L_2$ = aryloxy, C1-C18 alkyloxy, C5-C7 cycloalkyl, C1-C18 alkyl, C1-C18 alkenyl or tertiary C1-C18 alkyl amine.

**9.** The contrast agent particle of any one of claims 6 to 8, **characterized in that** n = 0 or 1.

**10.** The contrast agent particle any one of claims 3 to 9, **characterized in that** the linker is anchored to C4 of one of the one or more aryl group(s) of the arylated bismuth compound.

**11.** The contrast agent particle of any one claims 1 to 10, **characterized in that** the carrier particle is a silica particle or is composed of a biocompatible polymer selected from the group consisting of alginates, chitosans, dextrans, chitins, celluloses, polyethylenglycols (PEG), polylactic acids (PLA), polyglycolic acid (PGA), poly-3-hydroxybutyrate (PHB), poly(lactic-co-glycolic acid) (PLGA) and polycaprolacton (PCL) and derivatives thereof.

**12.** The contrast agent particle of any one of claims 1 to 11, **characterized in that** it comprises a targeting species attached to the carrier particle, wherein the targeting species is preferably selected from the group consisting of (recombinant) proteins and fragments thereof, peptides, peptidomimentic compounds, oligonucleotides, small organic molecules, peptide nucleic acids, sugars, folate, porphyrin derivatives, and combinations thereof.

**13.** The contrast agent particle of any one of claims 1 to 12 as an Magnetic Resonance Imaging (MRI) contrast agent

in Magnetic Resonance Imaging (MRI).

**14.** A Magnetic Resonance Imaging contrast medium (MRI contrast medium) comprising a contrast agent particle according to any one of claims 1 to 13.

**15.** The MRI contrast medium according to claim 14, further comprising a pharmaceutically acceptable carrier and/or excipient.

**16.** The MRI contrast medium according to claim 14 or 15, **characterized in that** it is administration of contrast agents into body tissues and/or body cavities/orifices.

**17.** Use of a contrast agent particle of any one of claims 1 to 13 or of a Magnetic Resonance Imaging contrast medium of any one of claims 14 to 16 in Quadrupole Relaxation Enhancement (QRE)-based Magnetic Resonance Imaging.

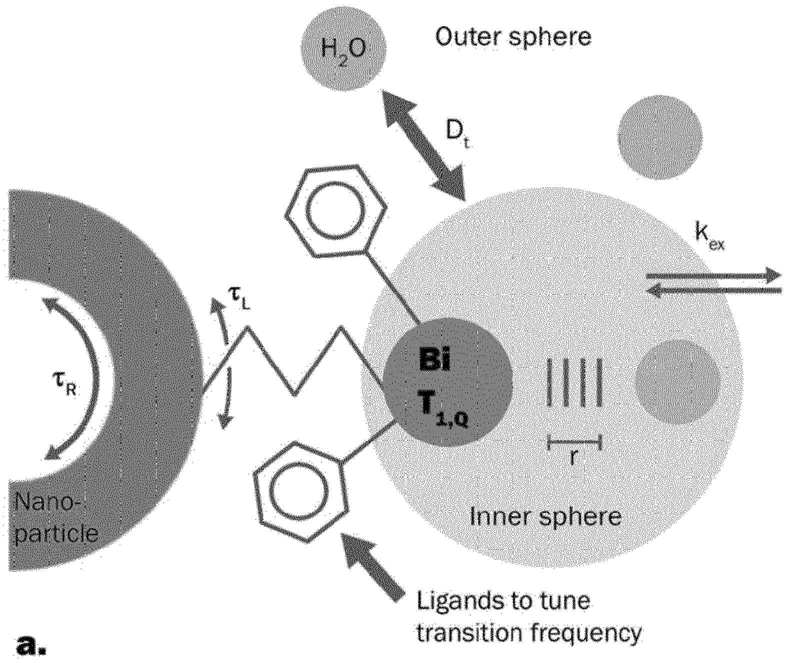


Fig. 1

Schematic view of the structure and the action mechanism of the new CAs when containing $^{209}$Bi as the QN: Water molecules approach the Bi atom by translational diffusion ($D_t$). Protons relax with shortened T1 (at specific frequencies) due to mutual dipole-dipole coupling to QN. $k_{ex}$ symbolizes the water exchange ratio, r is the distance between proton and $^{209}$Bi. The $^{209}$Bi -atom possesses ligands which, together with the external static magnetic field determine its quadrupole resonance transition frequencies. Bi and the ligands constitute the core compound, **component (i)**. Component 1 is grafted via a linker (zig-zag line) onto a biocompatible nanoparticle. The linker forms **component (ii)** while the nanoparticle represents c**omponent (iii)**.

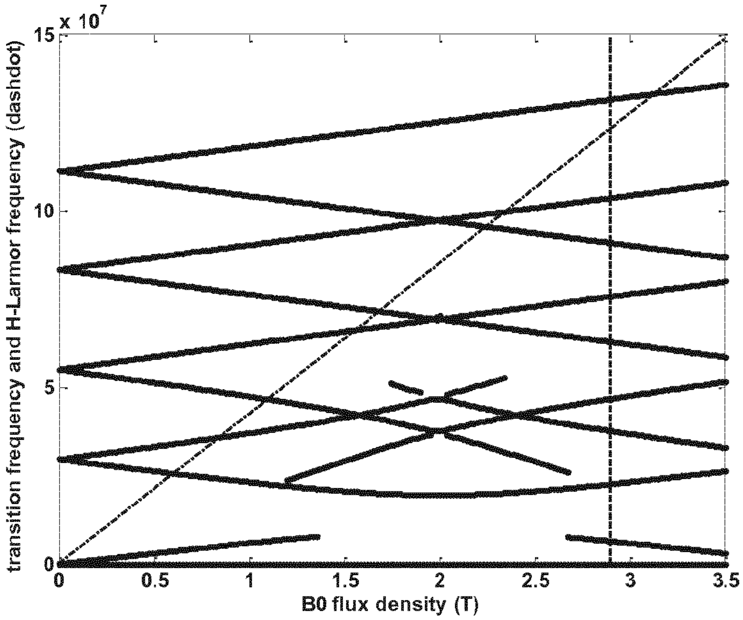


Fig. 2

Transition frequencies of $BiPh_3$ (solid curves) and proton Larmor frequency $f_0$ (dashdot) as a function of the magnetic field $B_0$, in case of mono-crystals with the EFG main axis aligned with $B_0$. The crossing points between the solid curves and the dashdotted line mark frequencies close to which the QRE occurs, if molecular dynamics allows for the process. Here the crossings occur at 25.5, 36.0, 47.2, 66.7, 71.9, 95.9, 99.7 and 133.4 MHz. The dashed vertical line denotes the $B_0$ of a clinical SIEMENS Skyra MRI scanner with an effective field of 2.89T. The targeted proton Larmor frequency $f_0$ is thus 123.4 MHz.

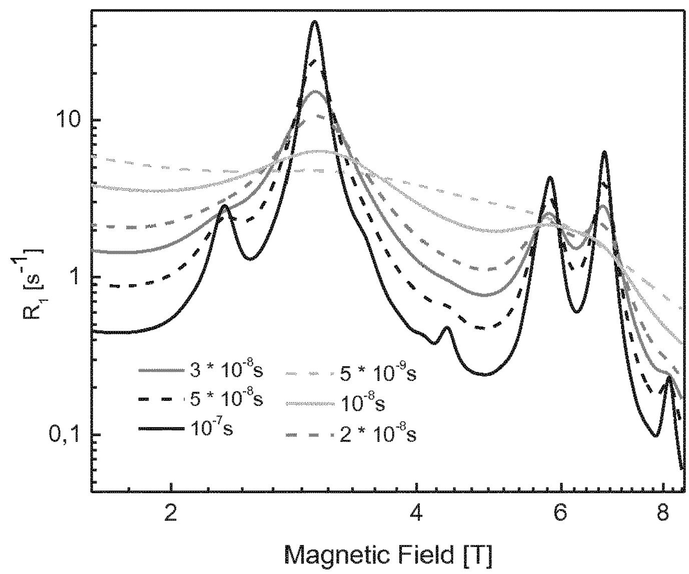


Fig. 3

Influence of the rotational correlation time $\tau_R$ on the simulated $R_1$. Fluctuations of EFG are not included.

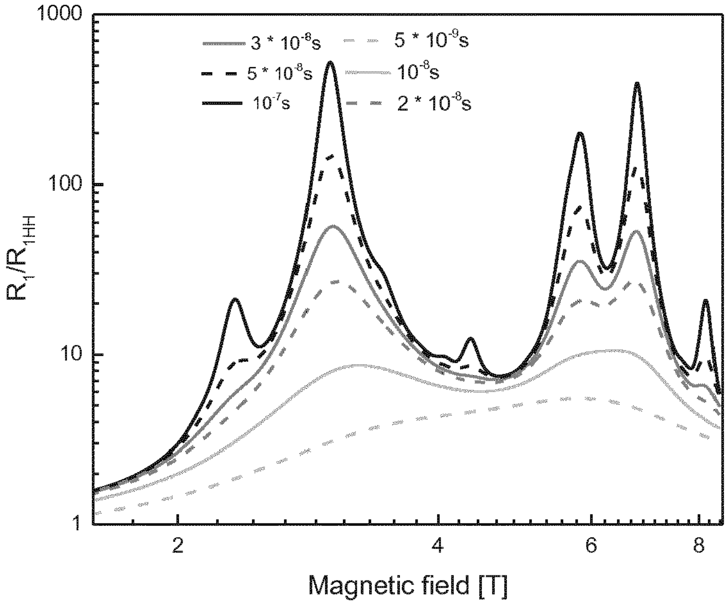


Fig. 4

Ratio between the relaxation rate $R_1$ caused by $^1H$-$^{209}Bi$ dipole-dipole interactions and the $R_{1HH}$ relaxation rate originating from proton-proton dipolar interactions for the same rotational correlation time and inter-spin distance.

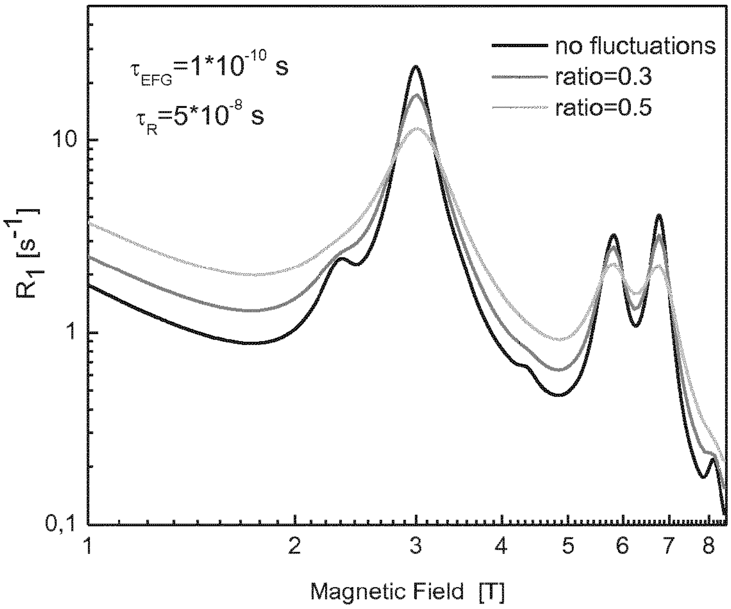


Fig. 5 Influence of the fluctuations of EFG on the simulated $R_1$

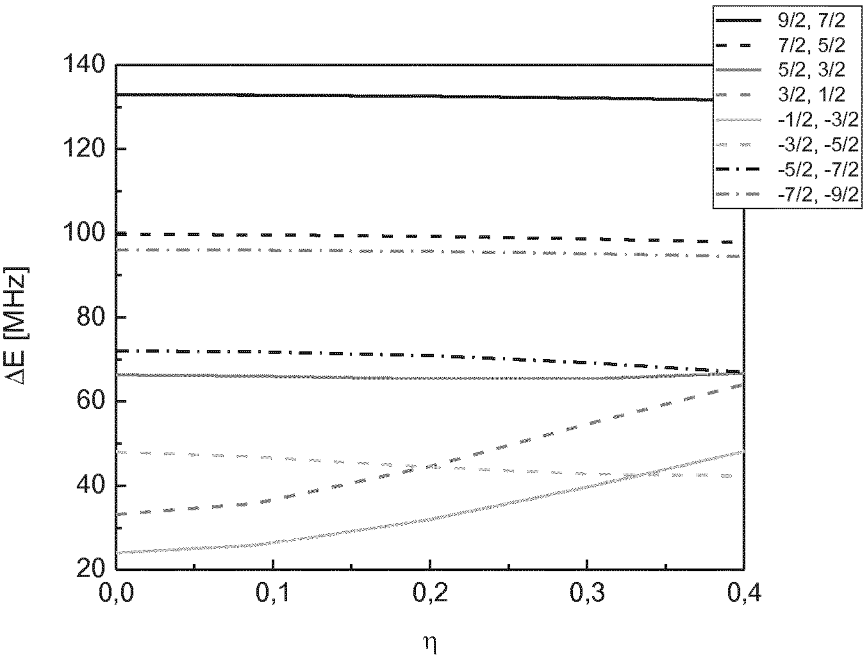


Fig. 6 Influence of η on $f_{Q,k}$

Europäisches Patentamt
European Patent Office
Office européen des brevets

# EUROPEAN SEARCH REPORT

Application Number
EP 16 18 8270

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X<br>Y | US 2010/297019 A1 (LANZA GREGORY M [US] ET AL) 25 November 2010 (2010-11-25)<br>* figures 1, 3 *<br>* paragraphs [0013], [0015], [0041], [0042], [0054], [0076], [0077], [0080], [0105] *<br>* example 2 *<br>----- | 1,2, 11-16<br>3-10,17 | INV.<br>A61K49/08<br>A61K49/18 |
| X<br><br>Y | DORIS BREITWIESER ET AL: "Photoreductive generation of amorphous bismuth nanoparticles using polysaccharides - Bismuth-cellulose nanocomposites", CARBOHYDRATE POLYMERS., vol. 116, 16 June 2014 (2014-06-16), pages 261-266, XP055352754, GB ISSN: 0144-8617, DOI: 10.1016/j.carbpol.2014.06.017<br>* the whole document and in particular the abstract and Figure 1 *<br>----- | 1,3-5, 11,13,14<br><br>1-17 | |
| X<br><br>Y | JUN CHEN ET AL: "In vitro and in vivo CT imaging using bismuth sulfide modified with a highly biocompatible Pluronic F127", NANOTECHNOLOGY, IOP, BRISTOL, GB, vol. 25, no. 29, 3 July 2014 (2014-07-03), page 295103, XP020267646, ISSN: 0957-4484, DOI: 10.1088/0957-4484/25/29/295103 [retrieved on 2014-07-03]<br>* the whole document and in particular the abstract; page 3, sections 2.4, 2.8-2.10; Scheme 1 *<br>-----<br>-/-- | 1,11, 13-16<br><br>1-17 | TECHNICAL FIELDS SEARCHED (IPC)<br>A61K |

The present search report has been drawn up for all claims

1

| Place of search | Date of completion of the search | Examiner |
|---|---|---|
| The Hague | 13 March 2017 | Birikaki, Lemonia |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

Europäisches Patentamt
European Patent Office
Office européen des brevets

# EUROPEAN SEARCH REPORT

Application Number
EP 16 18 8270

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | L A ZEMNUKHOVA ET AL: "209Bi NQR study of bismuth (III) complexes", RUSSIAN CHEMICAL BULLETIN, vol. 47, no. 11, 1 November 1998 (1998-11-01), pages 2169-2172, XP055354017, * the whole document * ----- | 1-17 | |
| | | | TECHNICAL FIELDS SEARCHED (IPC) |

The present search report has been drawn up for all claims

1

| Place of search | Date of completion of the search | Examiner |
|---|---|---|
| The Hague | 13 March 2017 | Birikaki, Lemonia |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

# ANNEX TO THE EUROPEAN SEARCH REPORT ON EUROPEAN PATENT APPLICATION NO. EP 16 18 8270

This annex lists the patent family members relating to the patent documents cited in the above-mentioned European search report. The members are as contained in the European Patent Office EDP file on
The European Patent Office is in no way liable for these particulars which are merely given for the purpose of information.

13-03-2017

| Patent document cited in search report | Publication date | Patent family member(s) | Publication date |
|---|---|---|---|
| US 2010297019 A1 | 25-11-2010 | CN 101868180 A | 20-10-2010 |
| | | EP 2209420 A1 | 28-07-2010 |
| | | US 2010297019 A1 | 25-11-2010 |
| | | WO 2009049083 A1 | 16-04-2009 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 5817289 A


### Non-patent literature cited in the description


- **RABIN O. et al.** *Nat. Mater.,* February 2006, vol. 5 (2), 118-122
- *Journal of Organic Chemistry,* 2016, vol. 81, 5401-5416
- **KRUK et al.** *Solid State Nucl. Magn. Reson.,* vol. 40 (3), 114-120
- Understanding Spin Dynamics. CRC Press, 16 October 2015